# EUROPEAN PATENT APPLICATION

(11) **EP 0 849 274 A1**
(43) Date of publication of application: **24.06.1998**
(21) Application number: 96250295.1
(22) Date of filing: 20.12.1996
(51) Int. Cl.: C07J 1/00, C07J 31/00, C07J 51/00

(54) **Bissteroidal compounds and their use for the preparation of chiral complexes**

(71) Applicant: SCHERING AKTIENGESELLSCHAFT, 13342 Berlin (DE)
(72) Inventor: Mohr, Jörg-Torsten, Dr., 10555 Berlin (DE); Enev, Valentien, Dr., 10179 Berlin (DE); Ewers, Christian, Dr., 10318 Berlin (DE); Harre, Michael, Dr., 12161 Berlin (DE); Nickisch, Klaus, Dr., 12307 Berlin (DE)

(57) **Abstract**

Bissteroidal compounds of general formula I 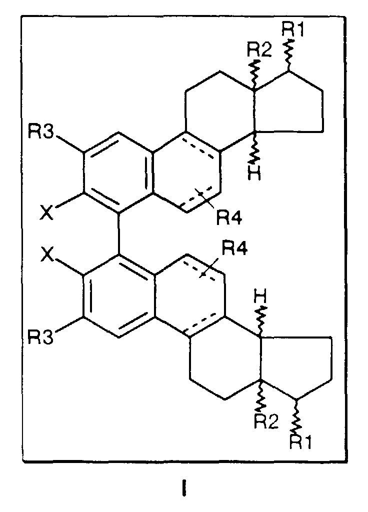 in which
- R₁: stands for hydrogen, alkyl, acyl, fluorine, and X₁R₅,
where X₁ stands for oxygen and sulfur and
R₅ can either be hydrogen, alkyl, aryl,
- R₂: stands for hydrogen and alkyl; the stereochemistry of C-13, C-14 and C-17 may either be α or β,
- X: stands for oxygen, trifluormethylsulfonyloxy, or (R₆)₂P,
where R₆ can be aryl, alkyl and cycloalkyl,
- R₃: stands for hydrogen, alkyl, aryl, trialkylsilyl, fluorine and X₂R₆,
where X₂ stands for oxygen and sulfur and
R₆ stands for hydrogen, trifluormethylsulfonyl, alkyl or aryl.
- R₄: can either be a substituent in the 6 or 7 position of the steroid with the meaning of hydrogen, alkyl, aryl, fluorine, and X₃R₇,
where X₃ stands for oxygen, sulfur or trialkylsilyl and R₇ stands for hydrogen, trifluormethylsulfonyl, alkyl or aryl,
and the B-ring of the steroid contains none or two double bonds.
Enantio- and Diastereomeric derivatives of compounds of general formula I, metal complexes thereof and their use in enantioselective reactions.

## Description

The invention relates to bissteroidal compounds and their use for the preparation of chiral complexes, useful as catalysts for various asymmetric syntheses.

### Background of the invention

Chiral 2,2'-bis:diphenylphosphino-1,1'bi-2-naphtol (BINAP) introduced by Noyori in 1980¹ has found wide application as a chiral ligand in asymmetric catalysis from laboratory up to industrial scale.
¹ Miyashita, A.; Yasuda, A.; Takaya, H.: Toriumi, K.: Ito, T.; Souchi, T.; Noyori, R. *J*. *Am*. *Chem*. *Soc*. **1980**, *102*, 7932.

The mayor drawbacks of the Noyori-BINAP synthesis are a bromination reaction at 240° to 320° C and a late resolution step of a BINAP precursor by crystallization with either camphorsulfonic acid or 2,3-O-dibenzoyltartaric acid as resolving agent, providing a overall low yield of the desired reagent.(Nachr. Chem. Techn. Lab. **1996**, *44*, 996).

A new procedure by Verhoeven et al² circumvents the tedious introduction of the diphenylphosphino group and provides a more simple preparation of BINAP via nickel catalyzed phosphinylation of the corresponding ditriflate of 1,1'-Bi-2-naphtol (BINOL).
²Dongwei, C.; Payack, J. F.; Verhoeven, T. R. United States Patent 5,399,771.

Nevertheless their improved synthesis of BINAP is still based on the resolution of BINOL with trans-1,2-Diaminocyclohexane as a resolving agent³.
³Takaya, H.; Mashima, K.; Koyano, K.; Yagi, M.; Kumobayashi, H.; Taketomi, T.; Akutagawa, S.; Noyori, R. *J*. *Org*. *Chem*. **1986**, *51*, 629.

Although steroids are potentially interesting compounds for asymmetric catalysis due to their rigid backbone the synthesis of 4,4'-bis(steroids) and any application thereof as chiral ligands in asymmetric catalysis is unprecedented so far.

The present invention describes the diastereoselective synthesis of (Ra)- and (Sa)-4,4'-bis(3-Diphenylphosphinoestra-1,3,5(10),6,8-pentaene) and the application of these new bisphosphines as chiral ligands in metal complexes for the enantioselective hydrogenation of β-ketoesters,α,β-unsaturated acids and dehydroaminoacids.

### Detailed description of the invention

bissteroidal compounds of general formula I 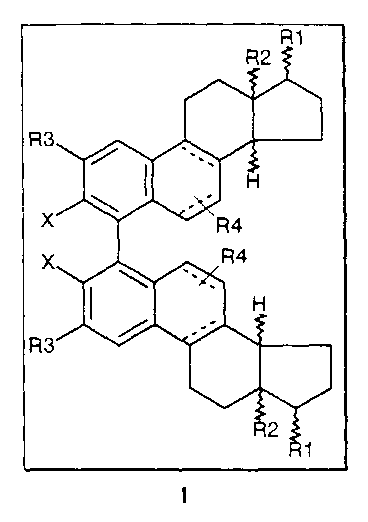 in which
- R₁: stands for hydrogen, alkyl, acyl, fluorine, and X₁R₅,
where X₁ stands for oxygen and sulfur and
R₅ can either be hydrogen, alkyl, aryl,
- R₂: stands for hydrogen and alkyl; the stereochemistry of C-13, C-14 and C-17 may either be α or β,
- X: stands for oxygen, trifluormethylsulfonyloxy,
or (R₆)₂P,
where R₆ can be aryl, alkyl and cycloalkyl,
- R₃: stands for hydrogen, alkyl, aryl, trialkylsilyl, fluorine and X₂R₆,
where X₂ stands for oxygen and sulfur and
R₆ stands for hydrogen, trifluormethylsulfonyl, alkyl or aryl.
- R₄: can either be a substituent in the 6 or 7 position of the steroid with the meaning of hydrogen, alkyl, aryl, fluorine, and X₃R₇,
where X₃ stands for oxygen, sulfur or trialkylsilyl and R₇ stands for hydrogen, trifluormethylsulfonyl, alkyl or aryl,
and the B-ring of the steroid contains none or two double bonds.

Enantio- and Diastereomeric derivatives of compounds of general formula I.

The alkyl radical of R₁ - R₇ have the meaning of lower alkyl substituents, per example the methyl-, ethyl-, propyl-, 2-methylethyl-, 2-methyl-propyl-, 3-methyl-propyl-, 2,2-dimethyl-ethyl- or butyl group. The acyl radical of R₁ have the meaning of C1-C6-groups, per example acyl-, propionylic-, butyric or hexanoic group. The aryl group radical of R₃, R₄, R₅, R₆ and R₇ have the meaning of phenyl-, benzyl- or 4-methyl-phenyl substituents. The cycloalkyl radical of R₆ has the meaning of a cyclopentyl- or cyclohexyl group. The trialkylsilyl radical of R3 or X3 have the meaning of trimethyl- or tert.-butyldimethylsilyl groups.

3-Hydroxy-1,3,5(10)6,8-estrapentaen-17-one (Equilenin) **1** is submitted to the standard conditions of a Wolff-Kishner reduction⁴ giving Estra-1,3,5(10),6,8-pentaen-3-ol **2** in 80-85% yield. 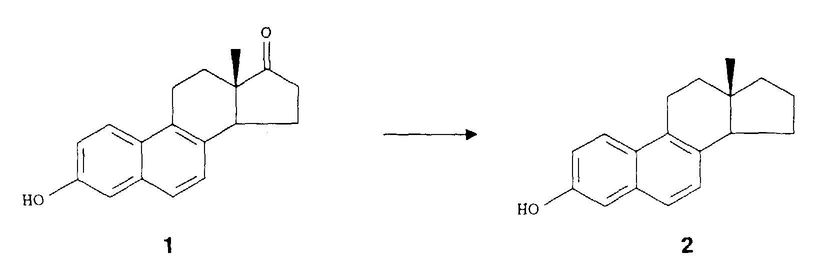
⁴Tietze, L. F.; Eicher, Th. Reaktionen und Synthesen, Thieme Verlag Stuttgart 1991.

The targets (Ra)- and (Sa)-4,4'-bis(Estra-1,3,5(10),6,8-pentaen-3-ol) (bisequilenol) **3** are prepared from Estra-1,3,5(10),6,8-pentaen-3-ol **2** via metal catalyzed phenolic coupling.

The coupling is performed with catalytic amounts of a copper-amine complex in methylenechloride under an oxygen atmosphere⁵ generating the diastereomers **S-3** and **R-3**. In contrast to the Noyori's BINOL synthesis these two diastereomers can be easily separated by flash chromatography thus avoiding the lengthy resolution of enantiomers with an additional resolution agent.
⁵Noji, M.; Nakajima, M.; Koga, K. *Tetrahedron Lett*. **1994**, *35*, 7983.

The absolute configuration of the new ligands (Ra)- and (Sa)-4,4'-bis(Estra-1,3,5(10),6,8-pentaen-3-ol) **3**, (Ra)- and (Sa)-4,4'-bis(Estra-1,3,5(10)-trien-3-ol) **5** and (Ra)- and (Sa)-4,4'-bis(3-diphenylphosphinoestra-1,3,5(10),6,8-pentaene) **7** were determined by CD spectroscopy in comparison to the CD spectra of the ligand (Ra)-bis(17-beta-Methoxyestra-1,3,5(10)-trien-3-ol) R-**4** whose absolute configuration was determined by X-ray crystallography.

Furthermore the diastereoselectivity of the coupling shows a temperature dependance favouring the R-isomer at room temperature with a diasteromeric excess of 50%. At lower temperature the selectivity is inverted, favouring the S-isomer with almost the same diasteromeric excess (Table 1).

In order to make both isomers available the coupling is performed at 0° C providing both diastereomers in a combined yield of 92-96%.

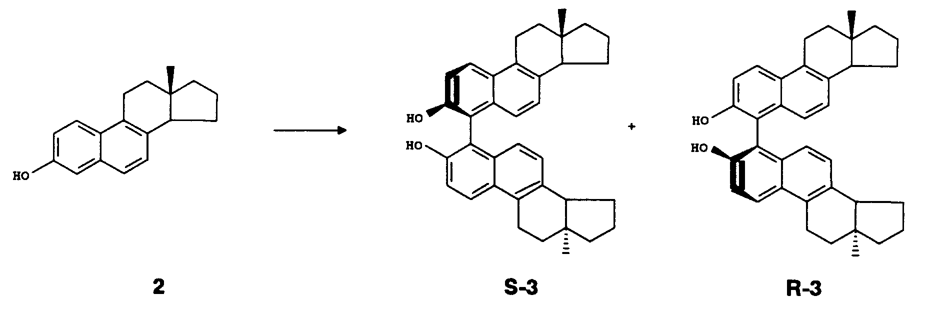

**Table 1**

| Dependence of the diasteroselectivity of the copper mediated coupling on the temperature | | |
|---|---|---|
| Temperature (° C) | S-3 | R-3 |
| + 20 | 1 | 3 |
| 0 | 1 | 1,5 |
| -20 | 3 | 1 |

The intriguing feature of these new compounds **R-3** and **S-3** is that they behave as true enantiomers in any type of asymmetric reactions despite of their diastereomeric nature. This was first shown testing the set of bissteroids **3-5** in enantioselective reductions of acetophenone following the Noyori protocol⁶ (Table 2).

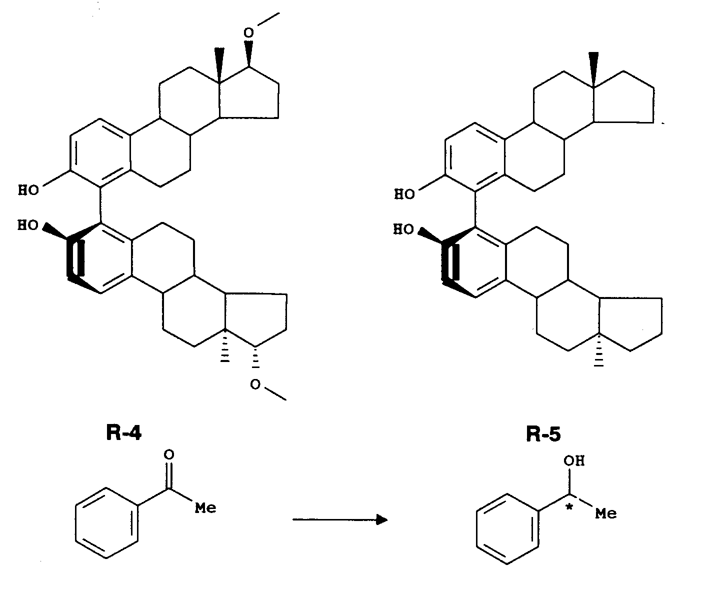

**Table 2**

| Enantioselective reductions of acetophenone induced by bissteroid ligands ⁶Noyori, R.; Tomino, I.; Tanimoto, Y. *J*. *Am*. *Chem*. *Soc*. **1979**, *101*, 3129. | | | |
|---|---|---|---|
| Ligand | %ee/Configuration | Temperature (°C) | Conversion (%)^{a} |
| R-3 | 92,5 / R | - 45 | > 95 |
| S-3 | 67 / S | -45 | > 95 |
| R-4 | 96,5 / R | -90 | > 95 |
| R-5 | 85,2 / R | -50 -30 | > 95 |

| | | | |
|---|---|---|---|
| a) determined by GC | | | |

The results in table 2 show that the new bissteroids are as powerful as reducing agents as Noyori's BINOL. The lower enantiomeric excess for the ligands **3** and **5** are due to solubility problems at very low temperatures which prevent the reduction to be carried out at -90° C as in the Noyori protocol. With ligand **4**, which is completely soluble at -90° C we achieve the same enantiomeric excess in the reduction of acetophenone as Noyori.

The absolute configuration of the reduction product is also the same as in the Noyori reduction.

As expected the reduction with the two diasteromeric ligands **R-3** and **S-3** produce the opposite enantiomers of the reduction product.

The bissteroids **R-/S-4** and **R-/S-5** were prepared by copper mediated coupling⁷ of the lithiated 4-Bromo-17 beta-methoxy-3-methoxymethoxyestra-1,3,5(10)-triene⁸ **11** and 4-Bromo-3-methoxymethoxyestra-1,3,5(10)-triene **14**.

The synthesis of (Ra)- and (Sa)-4,4'-bis(3-Trifluoromethylsulfonyloxyestra-1,3,5(10),6,8-pentaene) **6** was accomplished by triflation of **R-/S-3** with triflic acid anhydride in the presence of pyridine in a quantitative yield.

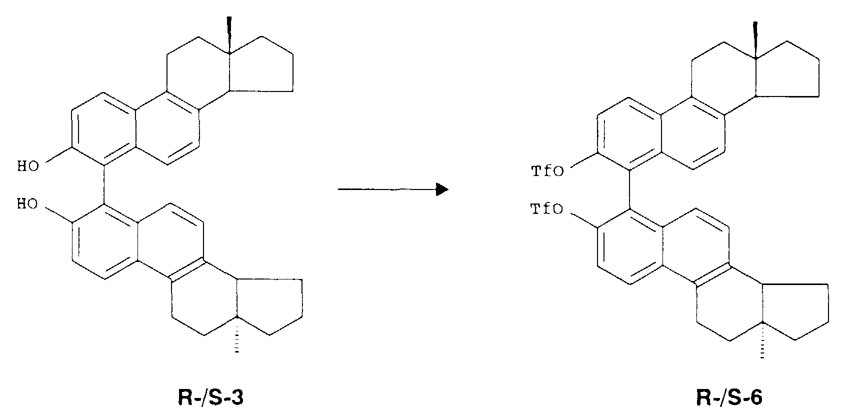

The triflate **6** was then converted into the bisphoshine **7** without any isomerisation of the diastereomers following the Merck protocol in 83% yield. 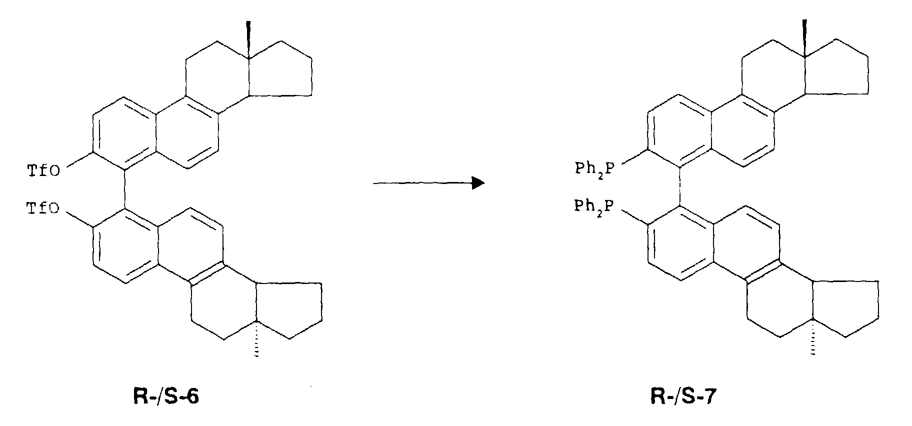
⁷Lipshutz, B. H.; Siegmann, K.; Garcia, E. *Tetrahedron* 1992, 48, 2579.
⁸Page, P. C. B.; Hussain, F.; Bonham, N. M.; Morgan, P.; Maggs, J. L.; Park, B. K. *Tetrahedron* **1991**, *47*, 2871.

Starting with Equilinin **1** both bisphosphines **R-7** and **S-7** are available in 4 chemical steps in an overall yield of 64%.

Both new phosphines **R-7** and **S-7** were tested as chiral ligands in a ruthenium complex for the asymmetric hydrogenation of Methyl-acetoacetate, N-Acetyl-cinnamic acid and tiglic acid as representative examples (Table 3).

The chiral ruthenium complex is prepared by heating the bisphosphines **R-/S-7** with [RuCl₂(benzene)]₂ in DMF.⁹

**Table 3**

| Asymmetric hydrogenation of Methyl-acetoacetate and N-Acetyl-cinnamic acid | | | | |
|---|---|---|---|---|
| Substrate | **R-7** ( %ee) | **S-7** (%ee) | conversion(yield) | **BINAP**(conv%) |
| Methyl-acetoacetate | > 99* | > 99* | 100(98) | >98(100)* |
| N-acetyl-cinnamic acid | 85,7** | 85,7** | 100(96) | 77(90)**** |
| Tiglic acid | 90,5*** | 90,5*** | >96(92) | 87,5(44)*** |

| | | | | |
|---|---|---|---|---|
| *1h/ 100 atm/ 100°C; | | | | |
| **48h/ 7atm; | | | | |
| ***24 h/ 4atm; | | | | |
| ****96h /7 atm; | | | | |

The new chiral complex synthesized from [RuCl₂(benzene)]₂ and the bissteroidal ligands **7** is more powerful as the state-of-the-art BINAP-ruthenium complexes of Noyori as it is of broader applicability and allows the preparation of a range of substrates of extraordinary high enantiomeric purity in a quantitative yield.

In addition the new bissteroidal ligands **3-5** which are intermediates in the synthesis of the bisphenylphosphine ligands have proven to be highly effective for the enantioselektive reduction of ketones exemplified by the reduction of acetophenone.

### Preparative Examples

Optical rotation were measured on a Perkin Elmer 241 Polarimeter, melting points are not corrected; ¹H-NMR (300 MHz), and ¹³C-NMR (75 MHz) spectra were taken on General Electric QE 300 in CDCl₃ as a solvent and internal reference (7.28 ppm, 81.9 ppm); J values are given in Hz; IR spectra were taken on Nicolet 20 SBX; Mass spectra were recorded on TRIO2; TLC were performed on Merck 60 F 254. Silica gel 60G (240-400 mesh) was used for column chromatography. All reactions were carried out under dry nitrogen or argon.
⁹Kitamura, M.; Tokunaga, M.; Ohkuma, T.; Noyori, R. *Tetrahedron Lett*. **1991**, *32*, 4163.

### Synthesis of (Ra)- and (Sa)-4,4'-bis(Estra-1,3,5(10),6,8-pentaen-3-ol) 3:

1. A suspension of 53,15 g 3-Hydroxy-1,3,5(10)6,8-estrapentaen-17-one **1** (0,199 mmoles) in 36,3 ml hydrazine hydrate, 435 ml diethyleneglycol and 36,5 g NaOH (0,91 mole) is heated on an oil bath to 120° C. After stirring at this temperature for 0,5 hour the temperature is increased to 180°-190° C and stirred for additional 8 hrs. After cooling to room temperature the solution is added to 500 ml of ice cooled water and then acidified with conc. HCl to pH 0. The precipitate is filtered off, dried and the crude product is recrystallized from water-ethanol 1:1 (100 ml each) giving 42,7 g Estra-1,3,5(10),6,8-pentaen-3-ol **2** (85%); mp = 136,5°-139° °C
2. To a solution of Estra-1,3,5(10),6,8-pentaen-3-ol **2** (10g, 20mmol ) in CH₂Cl₂ (300 ml) was added CuCl(OH)·TMEDA (0.800 g,0.35 mmol) and the solution was stirred at 0°C for 10-15 min while bubbling oxygen through the mixture. The reaction was monitored by TLC. At the end of the reaction HCl (10%, 5 ml) was added to the mixture and stirred for additional 15 min (the colour was changed from blue to yellow). The mixture was filtered and the residue was dried under vacuum to give crude product (5 g). From the filtrate after extraction with CH₂Cl₂ (3 X 10 ml) was isolated additionally 4.8 g product which was combined with the first fraction. The crude mixture was separated to give 5.9 g (Ra)-4,4'-bis(estra-1,3,5(10),6,8-pentaen-3-ol) **3** (59%) as man isomer; m.p. 207,4-207,5°C; [α] ₃₆₅ = + 303.13° (c=1.6 in THF);
   ¹H-NMR: 8.15 (1H, d, J=10.0Hz), 7.38 (1H, d, J=10.0Hz), 7.09 (1H, d, J=7.5Hz), 7.01 (1H, d, J=7.5 Hz), 4.98 (1H, s, OH), 0.70 (3H, s); ¹³C-NMR: 151.3 (s), 135.2 (s), 131.8 (s), 130.9 (s), 127.6 (s), 126.5 (d), 126.1 (d), 122.0 (d), 116.8 (d), 111.7 (s), 49.7 (d), 40.4 (s), 38.7 (t), 35.8 (t), 25.2 (t), 24.7 (t), 20.9 (t), 16.2 (q); MS-Cl: 520 (100, M⁺ + 1+ NH₃), 503 (90, M⁺); MS-El: 502 (100, M⁺), 389 (5), 235 (10), 195 (5), 157 (7);
   and 3.7 g (Sa)-4,4'-bis(Estra-1,3,5(10),6,8-pentaen-3-ol) **3** as minor isomer (37%), m.p. 287,0°C decomp. (Hexane) ; [α] ₃₆₅ = + 145.18° (c=1.4 in THF);
   ¹H-NMR: 8.10 (1H, d, J=9.0 Hz), 7.32 (1H, d, J=9.0 Hz), 7.05 (1H, d, J=6.0 Hz), 6.98 (1H, d, J=6.0 Hz), 5.10 (1H, s, OH), 0.70 (3H, s); ¹³C-NMR: 151.3 (s), 135.1 (s), 131.6 (s), 130.9 (s), 127.6 (s), 126.5 (d), 126.1 (d), 121.8 (d), 116.8 (d), 111.7 (s), 49.7 (d), 40.4 (s), 38.7 (t), 35.8 (t), 25.2 (t), 24.7 (t), 20.9 (t), 16.2 (q); MS-Cl: 520 (88, M⁺ + 1+ NH₃), 503 (100, M⁺); MS-El: 502 (100, M⁺), 389 (5), 251 (6), 235 (10), 195 (5), 157 (7);
3. To a solution of (Sa)-4,4'-bis(Estra-1,3,5(10),6,8-pentaen-3-ol) **3** (1.8 g, 3.6 mmol) in Toluene/Pyridine 15:1 (30 ml) was added over period of 30 min dropwise triflic anhydride and mixture was stirred for additional 30 min. The solution was diluted with water, the organic layer was washed with brine, dried with Na₂SO₄ and the solvent was evaporated. The residue was filtered through SiO₂ to yield crude product (2.8 g) which after crystallisation from hexane gave 2,5 g pure triflate (Sa)-4,4'-bis(3-Trifluoromethylsulfonyloxyestra-1,3,5(10),6,8-pentaene) **6** (90%), m.p.=168.2°C;[α] _{D}= - 97.6° (c= 1.7 in THF), ¹H-NMR: 8. 25 (1H, d, J=10.0 Hz), 7.58 (1H, d, J=10.0 Hz), 7.12 (1H, d, J=9.0 Hz), 7.01 (1H, d, J=9.0 Hz), 0.65 (3H, s); ¹³C-NMR: 144.3 (s), 138.5 (s), 131.6(s), 130.9 (s), 130.7 (s), 126.9(d), 126.6 (d), 124.3(d), 123.5 (s), 118.2 (d), 49.9 (d), 40.3 (s), 38.6 (t), 35.6 (t), 24.9 (t), 24.7 (t), 20.8 (t), 16.2 (q); ¹⁹F-NMR: 87.12; MS-Cl: 784 (3, M⁺ + 1+ NH₃), MS-El: 766 (100, M⁺), 633 (7) 483 (85), 389 (6), 309 (7),
   (Ra)-4,4'-bis(3-Trifluoromethylsulfonyloxyestra-1,3,5(10),6,8-pentaene) **6**: The compound was synthesised according to the same procedure as (Sa)-4,4'-bis(3-Trifluoromethylsulfonyloxyestra-1,3,5(10),6,8-pentaene) **6** to give after recrystallisation pure compound; m.p.=201.9°C. [α]_{D}= - 88.2° (c= 1.0 in THF), ¹H-NMR: 8.25 (1H, d, J=10.0Hz), 7.59 (1H, d, J=10.0 Hz), 7.12 (1H, d, J=9.0 Hz), 7.01 (1H, d, J=9.0 Hz), 0.66 (3H, s); ¹³C-NMR: 144.3 (s), 138.5 (s), 131.6 (s), 131.0 (s), 130.7 (s), 126.9 (d), 126.8 (d), 124.3 (d), 123.5 (s), 118.2 (d), 49.9 (d), 40.3(s), 38.6 (t), 35.6 (t), 24.9 (t), 24.7 (t), 20.8 (t), 16.2 (q); ¹⁹F-NMR: 87.12; MS-Cl: 784 (8, M⁺ + 1+ NH₃), MS-El: 766 (100, M⁺), 633 (7) 483 (75), 389 (6), 309 (7),
4. To solution of NiCl₂dppe (0.070 g, 0.13 mmol) in dimethyl acetamide (2 ml) was added diphenylphosphine (0.13 ml, 0.75 mmol) at room temperature, and the solution was heated to 100°C. After 45 min, a solution of triflate (Ra)- or (Sa)-4,4'-bis(3-Trifluoromethylsulfonyloxyestra-1,3,5(10),6,8-pentaene) (1g,1.3mmol) and 1,4-diazabicyclo[2.2.2]octane (0.62 g, 5.5 mmol) in dimethyl acetamide (4 ml) was added at once, the resulting green solution was kept at 100°C and three additional portions of diphenylphosphine (3 X 0.13 ml) were added at 1, 3 and 5 h later. The reaction was kept at 100°C for six days and then the dark brown solution was diluted with MeOH. The desired product was filtered and the filter cake was washed with MeOH and dried under vacuum. The crude product (0.820 g, 80%) was recrystallised from MeOH/Tol 10:1 to give 0.78 g pure phosphine
   (Ra)-4,4'-bis(3-Diphenylphosphinoestra-1,3,5(10),6,8-pentaene) **7**: (70%), m.p. °C, ¹H-NMR: 8. 05 (1H, d, J=10.0 Hz), 7.42 (1H, brd, J=10.0 Hz), 7.3-6.9 (10H, m) 6.50 (2H, ABq, J=8.0 Hz), 3.30 (2H, brd, J=8.5Hz), 2.63 (1H, dd, J= 5.1, 11.0 Hz), 2.2-1.3 (7H, m), 0.70 (3H, s); ¹³C-NMR: 145.8 (s), 138.8 (s), 137.4 (s), 133.6 (s), 132.6 (s), 131.6 (s), 130.6 (s), 130.1 128.3, 128.2, 127.6, 125.7, 124.9, 123.3, 50.3 (d), (each d), 40.7 (s), 39.0 (t), 36.1 (t), 25.4 (t), 24.9 (t), 21.2 (t), 16.8 (q); ³¹P-NMR: -15.42 (s); MS-Cl: 784 (8, M⁺ + 1+ NH₃), MS-El: 766 (100, M⁺), 633 (7) 483 (75), 389 (6), 309 (7),
   (Sa)-4,4'-bis(3-Diphenylphosphinoestra-1,3,5(10),6,8-pentaene) **7**: (70%), m.p. °C, α _{D} = - 97.6° (c = 0.17 in THF), ¹H-NMR: 7.92 (1H, d, J=10.0 Hz), 7.39 (1H, brd, J=10.0 Hz), 7.3-6.9 (10H, m), 6.75 (2H, ABq, J=8.0 Hz), 3.38 (1H, dd, 5.6, 21.0 Hz), 3.22 (1H, m), 2.72 (1H, dd, 6.5, 11.6 Hz), 2.12 (2H, m), 1.9-1.3 (5H, m), 0.55 (3H, s); ¹³C-NMR: 146.2 (s), 137.6 (s), 133.5 (s), 133.1 (s), 1325 (s), 131.8 (s) 131.5 (s), 130.0 (d), 129.9 (s), 127.6 ,127.5, 126.9, 125.5, 124.8, 122.9 50.0 (each d), 40.3 (s), 38.7 (t), 35.8 (t), 25.0 (t), 24.6 (t), 20.8 (t), 16.4 (q); ³¹P-NMR: -14.42 (s); MS-Cl: 784 (8, M⁺ + 1+ NH₃), MS-El: 766 (100, M⁺), 633 (7) 483 (75), 389 (6), 309 (7),

### Scheme for the synthesis of (Ra)-bis(17 beta-Methoxyestra-1,3,5(10)-trien-3-ol) 4 and (Ra)-4,4'-bis(Estra-1,3,5(10)-trien-3-ol) 5

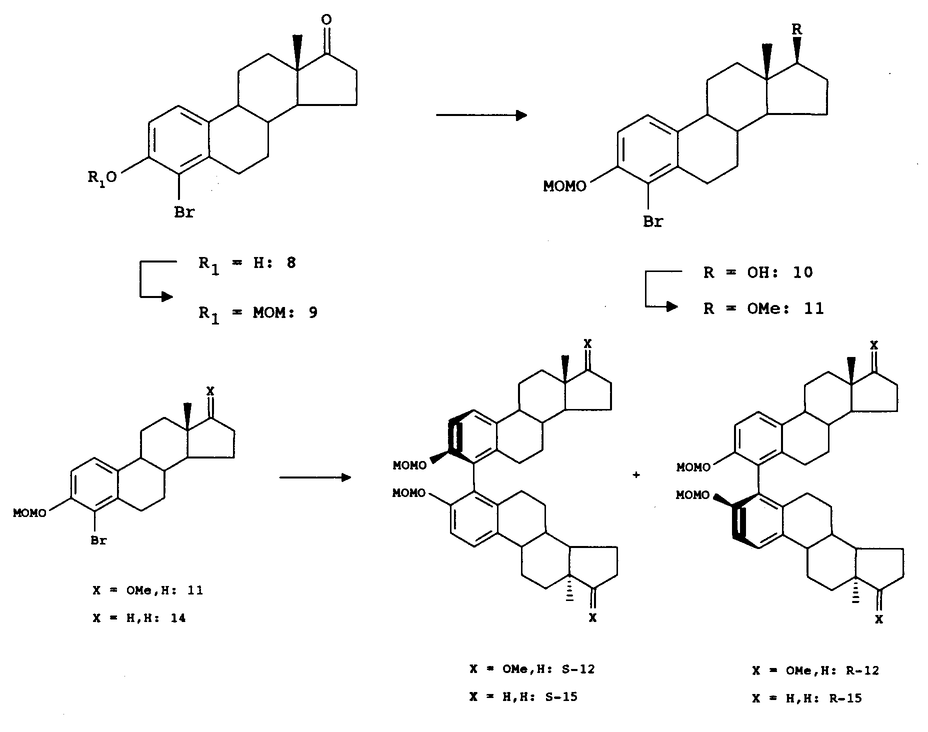

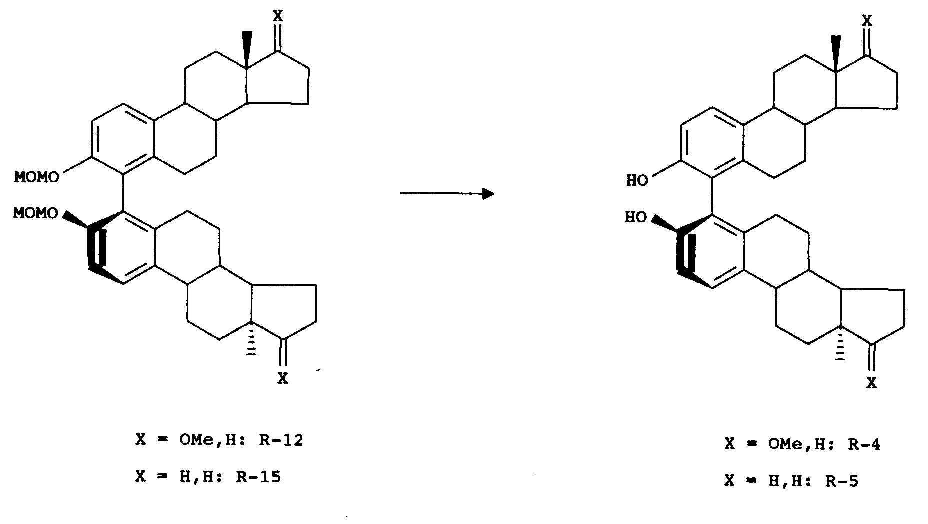

### Synthesis of (Ra)-bis(17 beta-Methoxyestra-1,3,5(10)-trien-3-ol) 4:

1. To solution of 4-Bromo-3-hydroxyestra-1,3,5(10)-trien-17-one **8** (20g, 51 mmol) in dimethyl formamide (250 ml) was added t-BuOK (7.7g, 74 mmol) at 0°C. The mixture was stirred for 5 min and then a solution methoxymethyl chloride (5.5 g, 68 mmol) in dimethyl formamide (15 ml) was added dropwise at the same temperature and stirred for additional 1 h. The reaction was quenched with saturated NH₄Cl and diluted with water (130 ml). The crude product was filtered and recrystalised from ethanol to give 21.5 g pure 4-Bromo-3-methoxymethoxyestra-1,3,5(10)-trien-17-one **9** (95.5%); m.p. 172° ;
2. The ketone 4-Bromo-3-methoxymethoxyestra-1,3,5(10)-trien-17-one **9** (20 g) was reduced with 10,6 g NaBH₄ (15 mmoles) in 300 ml methanol to give the alcohol 4-Bromo-3-methoxymethoxyestra-1,3,5(10)-trien-17-beta-ol **10** (20g) which was used without purification for the next step.
3. To solution of 4-Bromo-3-methoxymethoxyestra-1,3,5(10)-trien-17 beta-ol **10** (20g, 50 mmol) in DMF (250 ml) was added t-BuOK (7.9 g, 75 mmol) at 0°C. The mixture was stirred for 5 min and then a solution MeJ (9.2 g, 65 mmol) in dimethyl formamide (15 ml) was added dropwise at the same temperature and stirred for additional 15 h. The reaction was quenched with saturated NH₄Cl and diluted with water (130 ml). The crude product was filtered and recrystallized from ethanol to yield 19.7 g 4-Bromo-17 beta-methoxy-3-methoxymethoxyestra-1,3,5(10)-trien **11** (94%); [α] ₃₆₅ = + 49,6° (c=1 in THF); ¹H-NMR: 7.18 (1H, d, J=9.0 Hz), 6.95 (1H, d, J=9.0 Hz), 5.22 (2H, s), 3.52 (3H, s), 3.48 (3H, s), 3.30 (1H, t, J=7.5 Hz), 2.98 (1H, dd, J=5.8, 19.5 Hz), 2.70 (1H, m), 0.75 (3H, s);
   ¹³C-NMR: 151.3 (s), 137.3 (s), 135.8 (s), 124.4 (d), 115.9 (s), 113.0 (d), 94.9 (t), 90.4 (d), 57.4 (d), 55.9 (s), 49.9 (d), 43.8 (d), 42.8 (s), 37.6 (t), 37.4 (d), 30.8 (t), 27.4 (t), 27.1 (t), 26.3 (t), 22.7 (t), 11.1 (q); Anal. Calcd. (C₂₁H₂₉O₃Br) C-61.75, H-7.16, O-11.76, Br-19.52; Found: C-60.95, H-7.80, O-11.72, Br-1960;
4. To solution of 4-Bromo-17-beta-methoxy-3-methoxymethoxyestra-1,3,5(10)-trien **11** (10 g, 24.4 mmol) in THF (250 ml) was added BuLi (16 ml, 1.6 M, 26 mmol) at -60°C. The mixture was stirred for 30 min and then a suspension of CuCN (1.1 g, 12 mmol) in THF (25 ml) was added at the same temperature. The stirring was continued for additional 2.5 h until no CuCN was visible at the bottom of the flask. After cooling to -100°C dry oxygen was passed through the mixture for 15 min. which resulted in a deep purple colour. The oxygen flow was disconnected and the reaction mixture was stirred for additional 30 min at this temperature and then quenched with saturated NH₄Cl and diluted with diethyl ether (100 ml). The organic layer was washed with brine, dried with Na₂SO₄ and the solvent was evaporated. The crude mixture (7.8 g) was crystallized from ethyl acetate-hexanes 8:1. The solid product was filtered and dried to give 2.9 g of pure isomer (Ra)-4,4'-bis(17-beta-Methoxy-3-methoxymethoxyestra-1,3,5(10)-triene) **12** (36%). The filtrate was evaporated and the residue was separated by chromatography on SiO₂ (hexane:diethyl ether-6:1) to give additional product (0.2 g, 2%) which was combined with a first one and used without further purification.
   ¹H-NMR (crude): 7.28 (1H, d, J=9.0 Hz), 7.05 (1H, d, J=9.0 Hz), 5.09 (1H, d, J=6.0 Hz), 4.95 (1H, d, J=6.0 Hz), 3.48 (3H, s,), 3.30 (1H, t, J=8.5 Hz), 3.30 (3H, s), 0.80 (3H, s); ¹³C-NMR: 151.7 (s), 136.1 (s), 134.1 (s), 126.3 (s), 124.6 (d), 111.9 (d), 94.2 (t), 81.5 (d), 55.2 (d), 52.9 (s), 50.0 (d), 44.0 (d), 42.9 (t), 37.9 (d), 36.5 (t), 30.3 (t), 26.9 (t), 25.9 (t), 22.7 (t), 16.2 (q);
5. To solution of (Ra)-4,4'-bis(17-beta-Methoxy-3-methoxymethoxyestra-1,3,5(10)-triene) **12** (2g, 3 mmol) in Methanol/THF/H₂O 1:1:0.2 (20 ml) was added concentrated HCl (3ml) the mixture was stirred for 25 h at rt. The reaction mixture was neutralised with saturated NaHCO₃ and diluted with ethyl ether (50 ml). The organic layer was washed with brine, dried with Na₂SO₄ and the solvent was evaporated. The crude product (1.3 g, 78%) was recrystalised from Hexane ethyl ether 1:1 to give 1.1 g (Ra)-bis(17-beta-Methoxyestra-1,3,5(10)-trien-3-ol) **4** (70%); m.p. 181°C, [α] ₃₆₅ = - 96.30° (c=1.54 in THF); ¹H-NMR: 7.25 (1H, d, J=10.0 Hz), 6.82 (1H, d, J=10.0 Hz), 4.72 (1H, s, OH), 3.38 (3H, s), 3.32 (1H, t, J=7.5 Hz), , 0.80 (3H, s); ¹³C-NMR: 150.7 (s), 136.6 (s), 133.3 (s), 126.8 (d), 124.6 (s), 112.9 (d), 90.4 (d), 57.5 (d), 50.0 (s), 43.8 (d), 42.8 (t), 37.8 (d), 37.6 (t), 27.3 (t), 26.9 (t), 26.7 (t), 22.6 (t), 11.2 (q); Anal. Calcd. (C₃₈H₅₀O₄) C-79.96, H-8.83, O-11.21, Found: C-79.46, H-7.90, O-11.42,

### Synthesis of (Ra)-4,4'-bis(Estra-1,3,5(10)-trien-3-ol) 5:

1. To solution of 4-Bromo-3-hydroxy-estra-1,3,5(10)-trien (20g, 60 mmol) in dimethyl formamide (250 ml) was added t-BuOK (9.3g, 89 mmol) at 0°C. The mixture was stirred for 5 min and then a solution methoxymethyl chloride (6.3 g, 78 mmol) in DMF (15 ml) was added dropwise at the same temperature and stirred for additional 1 h. The reaction was quenched with saturated NH₄Cl and diluted with water (130 ml). The crude product was filtered and recrystalised from ethanol to give 20.4 g 4-Bromo-3-methoxymethoxy-estra-1,3,5(10)-trien **14** (90%); m.p. 85.2°C, [α]_{D} = + 50,8° (c=1 in THF); ¹H-NMR: 7.22 (1H, d, J=9.0 Hz), 6.97 (1H, d, J=9.0 Hz), 5.22 (2H, s,), 3.55 (3H, s,), 0.72 (3H, s); ¹³C-NMR: 151.3 (s), 135.2 (s), 131.8 (s), 130.9 (s), 127.6 (s), 126.5 (d), 94.2 (t), 81.5 (d), 55.2 (d), 52.9 (s), 50.0 (d), 44.0 (d), 42.9 (t), 37.9 (d), 36.5 (t), 30.3 (t), 26.9 (t), 25.9 (t), 22.7 (t), 16.2 (q); MS-Cl: 398, 396 (100, M⁺ + 1+ NH₃); MS-El: 380, 378 (30, M⁺), 350, 348 (25), 251 (6), 173 (10); Anal. Calcd. (C₂₀H₂₇O₂Br) C-63.33, H-7.17, O-8.44, Br-21.06; Found: C-63.28, H-7.20, O-8.44, Br-21.00;
2. To solution of 4-Bromo-3-methoxymethoxy-estra-1,3,5(10)-trien **14** (12 g, 31.7 mmol) in THF (250 ml) was added BuLi (24 ml, 1.6 M, 38 mmol) at -60°C. The mixture was stirred for 30 min and then a suspension of CuCN (1.5 g, 17mmol) in THF (25 ml) was added at the same temperature. The mixture was stirred for additional 2.5 h until no CuCN was visible at the bottom of the flask. After cooling to -100°C a dry oxygen was passed through the mixture for 15 min. The oxygen flow was disconnected and the reaction mixture was stirred for additional 30 min at this temperature and then quenched with saturated NH₄Cl and diluted with diethyl ether (100 ml). The organic layer was washed with brine, dried with Na₂SO₄ and the solvent was evaporated. The residue (10 g) was dissolved in DMF (30 ml) and to the solution was added t-BuOK (1.9 g, 18 mmol) at 0°C. The mixture was stirred for 5 min and then a solution TBDMSCl (2.5 g, 65 mmol) in DMF (15 ml) was added at the same temperature and stirred for additional 30 min. The reaction was quenched with saturated NH₄Cl and diluted with ethyl ether (50 ml). The organic layer was washed with brine, dried with Na₂SO₄ and the solvent was evaporated. The crude mixture (11 g) was separated by chromatography on SiO₂ (hexane:diethyl ether-6:1) to give 2 g of crystalline product (Ra)-4,4'-bis(3-Methoxymethoxyestra-1,3,5(10)-triene) **15** (21 %) which was used without purification for the next step.
3. To solution of (Ra)-4,4'-bis(3-Methoxymethoxyestra-1,3,5(10)-triene) **15** (2g, 3.3 mmol) in Methanol/THF/H₂O 1:1:0.2(20 ml) was added conc. HCL (3ml) and stirred for 25 h at room temperature. The reaction mixture was neutrlized with saturated NaHCO₃ and diluted with ethyl ether (50 ml). The organic layer was washed with brine, dried with Na₂SO₄ and the solvent was evaporated. The crude product (1.3 g, 78%) was recrystallised from hexane / ethyl ether 1:1 to give 1,3 g (Ra)-4,4'-bis(Estra-1,3,5(10)-trien-3-ol) **5** (76%), m.p.162.5°C, [α] ₃₆₅ = - 96.30° (c=1.54 in THF); ¹H-NMR: 7.25 (1H, d, J=10.0 Hz), 6.82 (1H, d, J=10.0 Hz), 4.72 (1H, brs, OH), 0.72 (3H, s); ¹³C-NMR: 150.8 (s), 136.7 (s), 133.6 (s), 126.8 (d), 111.8 (s), 112.3 (d), 53.3 (d), 43.9 (d), 40.7 (s), 40.2 (t), 38.5 (t), 38.3 (d), 27.6 (t), 27.1 (t), 26.4 (t), 24.8 (t), 20.2 (t), 14.8 (q); MS-Cl: 520 (100, M⁺ + 1+ NH₃), 503 (92, M⁺ + 1); MS-El: 520 (100, M⁺), 251 (4), 235 (6); Anal. Calcd. (C₃₆H₄₆O₂) C-84.66, H-9.08, O-6.26, Found: C-84.94 H-9.12, O-6.20;

### Examples of applications:

### 1. Reduction of acetophenone with ligand R-4:

A dry 50 ml Schlenk tube containing a Teflon coated stirring bar was charged with solution of LiAlH₄ in THF (1. 5 ml, 0.98 M) and then a solution of ethyl alkohol in THF (0.9 ml, 1.7 M) was added dropwise for a period of ca. 10 min. Subsequently a THF solution of **R-4** (3 ml, 0.53 M) was added dropwise for a period of 30 min. After stirring for additional 30 min. at room temperature the reducing agent was cooled to -90°C. A solution of acetophenone (0.45 ml, 0.7 M) in THF was added slowly (for a period of 20 min). The mixture was stirred for additional 3 h at this temperature and at -78°C for 16 h. After addition of methanol (0.5 ml) at -78°C the mixture was warmed up to room temperature, neutralysed to pH=6 with HCL (5%) and extracted with ethyl ether (2 X 10 ml). GC analysis (25 m FS-Hydrodex-B-PM, 110 isoterm, 1 bar Hydrogen, t° detector=280°C, t° column = 280°C) of the extract shows 96.5% ee and complete conversion.

### 2. Hydrogenation of β-Ketoesters with ligand R-4:

A dry 50 ml Schlenk tube containing a Teflon coated stirring bar was charged with [RuCl₂(benzene)]₂ (0.018 g, 0.036 mmol), **S-4** (0.07 g, 0.073 mmol) and DMF (1 ml). The resulting brown suspension was heated at 100°C under argon for 30 min to give a clear reddish brown solution. The reaction mixture was cooled and concentrated at 1 mm Hg and then at 0.05 mm Hg for 1h to give RuCl₂(S-4)(DMF)₂.

### a) Hydrogenation of methyl acetoacetate

To the resulting reddish brown solid of RuCl₂(S-4)(DMF)₂ was added a solution of methyl acetoacetate (7.3 g, 63 mmol) in degassed methanol (40 ml) and stirred for 5 min. Then the solution was transferred to 125 ml stainless steel autoclave and kept 1h at 100°C and under hydrogen (100 atm) and then 10 h at room temperature and same pressure. After the excess hydrogen had been blown off, the apparatus was disassembled. The content was concentrated. Distillation (110°C, 46 mmHg) afforded methyl (S)-3-hydroxybutanoate (7.22 g, 98%, [α]_{D}= - 50.5 c= 1.4, [α]₅₄₆ = - 58.3 c= 1.4 in CHCl₃), in 99% ee assayed as MTPA ester.

### b) Hydrogenation of acrylic acid:

To the resulting reddish brown solid of RuCl₂(S-4)(DMF)₂ was added a solution of α-acetamidocinnamic acid (3.6 g, 17 mmol) in degassed methanol (40 ml) and stirred for 5 min. Then the solution was transfered in to 125 ml stainless steel autoclave and kept 48h at room temperature and under hydrogen (4 atm). After the excess hydrogen had been blown off, the apparatus was disassembled. The content was concentrated to give 3.6 g crude product. A small amount from the crude product (0.05 g) was treated with excess of diazomethane (solution in ethyl ether) to yield the corresponding methyl ester for determination of the enantiomeric excess. GC analysis (25 m Chiralsil-DEX, 150° isoterm, 1 bar hydrogen, t° detector=280°C, t° colum = 280°C) of this ester shows an ee of 85.7% and no trace of the starting material. The rest of the product was dissolved in hot water (200 ml) and extracted with toluene (2X30 ml). The water was evaporated and the residue was dried under vacuum to give 3. 48 g (96%) (-)-N-acetyl-phenyl alanine, [α]_{D} = - 33.7 (c= 1.0 in methanol).

### c) Hydrogenation of tiglic acid:

To the resulting reddish brown solid of RuCl₂(S-4)(DMF)₂ was added a solution of tiglic acid (1.2 g, 17 mmol) in degased methanol (30 ml) and stirred for 5 min. Then the solution was transferred to 200 ml stainless steel autoclave and kept 24h at r.t and under hydrogen (4 atm). After the excess hydrogen had been blown off, the aparatus was disassembled. The content was concentrated to give 3.6 g crude product. A small amount from the crude product (0.05 g) was treated with exess of diazomethane (solution in ethyl ether). GC analysis (25 m Chiralsil-DEX, 150° isoterm, 1 bar Hydrogen, t° detector=280°C, t° colum = 280°C) of this solution shows 90.5 % ee and no trace of the starting material. Distillation (78°C, 24 mmHg) afforded methyl (R)-2-methylbutyric acid (1.15 g, 92%, [α]_{D} = -17.2 (neat).
¹⁰Shibasaki et al Angew. 1996, *108*, 2489.

## Claims

1. Bissteroidal compounds of general formula I 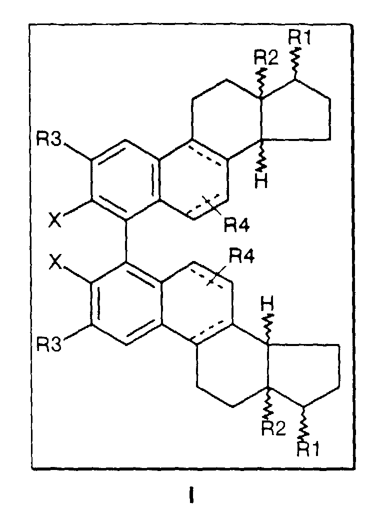 in which
R₁ stands for hydrogen, alkyl, acyl, fluorine, and X₁R₅,
where X₁ stands for oxygen and sulfur and
R₅ can either be hydrogen, alkyl, aryl,
R₂ stands for hydrogen and alkyl; the stereochemistry of C-13, C-14 and C-17 may either be α or β,
X stands for oxygen, trifluormethylsulfonyloxy, or (R₆)₂P,
where R₆ can be aryl, alkyl and cycloalkyl,
R₃ stands for hydrogen, alkyl, aryl, trialkylsilyl, fluorine and X₂R₆,
where X₂ stands for oxygen and sulfur and
R₆ stands for hydrogen, trifluormethylsulfonyl, alkyl or aryl.
R₄ can either be a substituent in the 6 or 7 position of the steroid with the meaning of hydrogen, alkyl, aryl, fluorine, and X₃R₇,
where X₃ stands for oxygen, sulfur or trialkylsilyl and R₇ stands for hydrogen, trifluormethylsulfonyl, alkyl or aryl,
and the B-ring of the steroid contains none or two double bonds.

2. Enantio- and Diastereomeric derivatives of compounds of general formula I.

3. Use of the compounds of claim 1 and 2 for the preparation of chiral metal complexes.

4. Use of the compounds of claim 1 and 2 for the preparation of chiral metal complexes contaning rhodium, ruthenium, palladium, osmium, iridium, platinum, zink, titanium, aluminum, gold, lanthanoids or mixed lanthanoid - alkalli complexes .

5. Use of the compounds of claim 1 and 2 for the preparation of chiral complexes containing rhodium, ruthenium, palladium, osmium, iridium, platiniumtitanium, aluminum, gold, lanthanoids or mixed lanthanoid - alkalli complexes for all classes of asymmetric reactions, especially for the enantioselective hydrogenation of alkenes, enamides, aldehydes or ketones, enantioselective Michael-addition reactions¹⁰, enantioselective Ene-reactions, enantioselective Alkylation of aldehydes and ketones , enantioselective Dils-Alder reactions and enantioselective Hydrosilylation.
